Europäisches Patentamt

European Patent Office

Office européen des brevets

(11) Veröffentlichungsnummer: **0 291 086 B1**

(12) **EUROPÄISCHE PATENTSCHRIFT**

(45) Veröffentlichungstag der Patentschrift: **13.04.94**

(21) Anmeldenummer: **88107755.6**

(22) Anmeldetag: **13.05.88**

(51) Int. Cl.⁵: **G01N 33/68**, G01N 33/96, //G01N33/563

(54) **Verfahren zur Bestimmung eines Antikörpers in menschlichen Körperflüssigkeiten.**

(30) Priorität: **14.05.87 DE 3716217**
**04.01.88 DE 3800048**

(43) Veröffentlichungstag der Anmeldung:
**17.11.88 Patentblatt  88/46**

(45) Bekanntmachung des Hinweises auf die Patenterteilung:
**13.04.94 Patentblatt  94/15**

(84) Benannte Vertragsstaaten:
**AT BE CH DE ES FR GB GR IT LI LU NL SE**

(56) Entgegenhaltungen:
**WO-A-86/01533**
**GB-A- 2 013 337**

**DIABETES, vol. 34, Januar 1985, New York, NY (US); L.J. NELL et al., Seiten 60-66&NUM;**

**DATABASE WPIL/DERWENT; Nr. 85-194635&NUM;**

**DATABASE WPIL/DERWENT; Nr. 88-011835&NUM;**

(73) Patentinhaber: **BOEHRINGER MANNHEIM GMBH**

**D-68298 Mannheim(DE)**

(72) Erfinder: **Kientsch-Engel, Rosemarie, Dr.**
**Moosstrasse 7**
**D-8121 Pähl(DE)**
Erfinder: **Wörner, Walter, Dr.**
**Kaltenmoserstrasse 14**
**D-8120 Weilheim(DE)**
Erfinder: **Kleinhammer, Gerd, Dr.**
**Kreuzeckstrasse 3**
**D-8132 Tutzing(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

ANNALES DE BIOLOGIE CLINIOUE, Band 48, Nr. 7, 1990, Paris (FR); R.G. HAMILTON, Seiten 473-477&NUM;

D.M. KEMENY et al. (eds.), "Elisa and other solid Phase immunoassays", 1988, John Wiley & Sons Ltd., New York, NY (US); Seiten 57-82&NUM;

**Beschreibung**

Die Erfindung betrifft ein Verfahren zur Bestimmung eines Antikörpers in menschlichen Körperflüssigkeiten nach dem Prinzip des Immunoassays, wobei man eine den zu bestimmenden Antikörper enthaltende Probe mit mindestens zwei verschiedenen Rezeptoren $R_1$, $R_2$, von denen ein Rezeptor $R_1$ eine für den zu bestimmenden Antikörper spezifische antigene Determinante trägt und ein Rezeptor $R_2$ eine Markierung trägt, inkubiert, den Teil, der die gebundene Markierung enthält von dem Teil, der die ungebundene Markierung trägt, trennt und die Markierung in einem der beiden Teile mißt und ein geeignetes Kontrollreagenz.

Die Bestimmung von in Körperflüssigkeiten vorhandenen Antikörpern ist oft wünschenswert. So gibt der Nachweis bestimmter Antikörper einen Hinweis, ob eine Infektion durchgemacht wurde oder ob eine Allergie besteht. Dies ist z. B. wichtig, wenn überprüft wird, ob bei Medikamentengabe mit einem allergisch bedingten Schock zu rechnen ist. Weiterhin kann durch Nachweis von Auto-Antikörpern eine Auto-Immunerkrankung diagnostiziert werden. Wichtig ist auch der Nachweis von durch Therapie z.B. mit Medikamenten, die rekombinante Proteine (z.B. Insulin, Erythropoietin) enthalten, gebildeten Antikörpern, wie z. B. Antiinsulin-Antikörpern.

Da die Antikörper im Blut in sehr geringen Mengen vorkommen, sind zu ihrem Nachweis sehr empfindliche Verfahren notwendig.

Daher bietet sich zu ihrem Nachweis ein Verfahren nach dem Prinzip des Immunoassays an. Ein Vorteil dieser Bestimmungsmethode ist ihre Genauigkeit und die Möglichkeit, sehr geringe Substanzmengen nachweisen zu können.

Zur Durchführung der Bestimmung sind verschiedene Verfahrensvarianten möglich sowohl in homogenen, als auch in heterogenen Phasen. Bei der Ausführungsform in heterogener Phase wird einer der Rezeptoren an einen Träger gebunden. Beispielsweise wird beim Sandwich-Verfahren ein eine für den zu bestimmenden Antikörper spezifische antigene Determinante tragender Rezeptor an den Träger gebunden, die Testlösung zugegeben, wobei der in der Testlösung enthaltene, zu bestimmende Antikörper an den Rezeptor gebunden wird. Dann wird ein markierter Rezeptor zugegeben, der spezifisch mit dem Antikörper oder dem Antigen-Antikörper-Komplex reagiert. Über den markierten Rezeptor kann dann die Menge an Antikörper in der Probe bestimmt werden. Für dieses allgemeine Prinzip gibt es viele Variationsmöglichkeiten. So kann beispielsweise eine Bestimmung mit drei Rezeptoren erfolgen, wobei einer der drei Rezeptoren in heterogener Phase vorliegt und die anderen beiden Rezeptoren löslich sind und wobei einer der beiden löslichen Rezeptoren markiert ist, während der andere unmarkiert ist. Der unlösliche Rezeptor ist dann gegen den nicht markierten löslichen Rezeptor gerichtet.

Die Bestimmung der Insulin-Antikörper erfolgt traditionell mit Radio-Immunoassay. Dabei wird die Patientenprobe mit jodmarkiertem Insulin inkubiert, das gebundene, radioaktive Insulin entweder mit Polyethylenglykol oder einem Zweitantikörper gegen menschliches Immunglobulin ausgefällt und die Radioaktivität des Präzipitats bestimmt. In letzter Zeit wurden auch Bestimmungsmethoden nach dem Prinzip des Enzym-Immunoassays beschrieben (T.J. Wilkin (1986), "The measurement of insulin antibodies and its interpretation" in Immunoassay Technology Vol. 2 (ec. S. B. Pal) W. de Gruyter, Berlin).

Nachteil dieser bekannten Verfahren ist es, daß sich Testergebnisse, die an verschiedenen Tagen, bei unterschiedlichen Reaktionszeiten mit dem Substrat oder unter unterschiedlichen Reaktionsbedingungen erhalten wurden, nicht vergleichen lassen. Deshalb wurde schon vorgeschlagen, ein Patientenserum, von dem bekannt ist, daß es Antikörper gegen Insulin enthält, oder Human-Immunglobulin, das aus einem positiven Patientenserum isoliert wurde, als internen Standard mitzuführen (B.M. Dean et al. (1986) Diabetologia 29, 339-342; Nell et al. (1985) Diabetes 34, 60-66)). Der Test des bekannt positiven Serums dient außerdem als Funktionskontrolle für alle zum Test eingesetzten Reagenzien. Das Problem bei der Verwendung von positiven Patientenserum ist jedoch, daß nicht jedes Labor Zugang zu entsprechenden Patientenseren hat und diese Patientenseren auch nicht in ausreichender Menge zur Verfügung stehen. Außerdem besteht die Gefahr, daß Patientenseren infektiös sein können, z. B. Hepatitis- oder HIV-Viren enthalten. Weiterhin bestehen große Schwankungen von Patient zu Patient, so daß Testergebnisse mit Standardseren von verschiedenen Patienten nicht vergleichbar sind. Selbst bei Seren, die zu verschiedenen Zeiten von demselben Patienten abgenommen wurden, ist mit unterschiedlichen Antikörpertitern zu rechnen. Eine Vergleichbarkeit von Werten, die von verschiedenen Labors erhalten wurden, besteht überhaupt nicht.

Es war daher Aufgabe der Erfindung, ein Verfahren zu schaffen, mit dem eine Funktionskontrolle von Bestimmungsverfahren zum Nachweis von Antikörpern möglich ist und die es weiterhin erlauben, Testergebnisse zu vergleichen.

Diese Aufgabe wird gelöst durch ein Verfahren zur Bestimmung eines Antikörpers in menschlichen Körperflüssigkeiten nach dem Prinzip des Immunoassays, wobei man eine den zu bestimmenden Antikörper enthaltende Probe mit mindestens zwei verschiedenen Rezeptoren $R_1$, $R_2$, von denen ein Rezeptor $R_1$ eine für den zu bestimmenden Antikörper spezifische antigene Determinante trägt und ein Rezeptor $R_2$ eine Markierung trägt, inkubiert, den Teil, der die gebundene Markierung enthält, von dem Teil, der die ungebundene Markierung enthält, trennt und die Markierung in einem der beiden Teile mißt, das dadurch gekennzeichnet ist, daß man zur Kontrolle statt der Probe eine Standardlösung einsetzt, die ein Konjugat aus einem mit dem für den zu bestimmenden Antikörper spezifischen Rezeptor $R_S$ bindefähigen Antikörper oder dessen Fab- oder F(ab')$_2$-Fragment und einem Human-Immunglobulin oder dessen Fc-Teil enthält.

Überraschenderweise wurde festgestellt, daß ein chemisch synthetisiertes Konjugat aus einem Antikörper, der mit dem für den zu bestimmenden Antikörper spezifischen Epitop bindefähig ist und einem unspezifischen Humanimmunglobulin genauso reagiert, wie der entsprechende Antikörper aus den Patientenproben. Dabei liefert der nicht-humane Antikörperanteil des Konjugats die spezifische Reaktion mit dem Rezeptor $R_S$, während das Human-Immunglobulin die Nachweisbarkeit gewährleistet. Das erfindungsgemäß verwendete Konjugat ist in unbeschränkter Menge verfügbar und kann immer wieder reproduzierbar hergestellt werden.

Verfahren zur Bestimmung eines Antikörpers in menschlichen Körperflüssigkeiten nach dem Prinzip des Immunoassays sind an sich bekannt. Diese Verfahren werden einerseits verwendet, um gegen Bakterien oder Viren gebildete Antikörper nachzuweisen. Auf diese Weise kann eine bereits erfolgte Infektion oder deren Verlauf verfolgt werden. Beispiele hierfür sind Antikörper gegen Hepatitis-Viren oder gegen HIV. Andererseits werden diese Verfahren verwendet, um Auto-Antikörper nachzuweisen. Auto-Antikörper sind gegen körpereigene Antigene gerichtete Antikörper, die bei Vorliegen einer Auto-Immunkrankheit gebildet werden. Hier dient der Nachweis des Auto-Antikörpers zur Diagnose der Krankheit. Beispiele sind Schilddrüsenantikörper oder die Auto-Antikörper gegen das eigene Insulin oder gegen Inselzellen des Pankreas, die wahrscheinlich mit dem Auftreten von Diabetes mellitus im Zusammenhang stehen. Wichtig ist auch der Nachweis von Insulin-Antikörpern, die bei manchen Diabetikern gebildet werden, die mit tierischem Insulin behandelt werden. Da das Insulin sich von Spezies zu Spezies nicht sehr unterscheidet, kommt es zu einer starken Kreuzreaktion, so daß durch Antikörper gegen irgendein Insulin das Insulin im Serum neutralisiert wird und gegebenenfalls sogar eine Insulinresistenz bedingen kann. Bei der Behandlung mit rekombinanten Proteinen (z.B. Erythropoietin oder Plasminogenaktivatoren) muß mit der Bildung von Antikörpern gegen diese Proteine gerechnet und dies ebenfalls geprüft werden. Weiterhin interessant für die Diagnose von Allergien ist der Nachweis von für bestimmte Allergene spezifischen Antikörpern.

Zur Durchführung des Verfahrens zur Bestimmung eines Antikörpers sind sehr viele Varianten möglich. Eine sehr weit verbreitete Variante ist der Sandwichassay, bei dem ein Rezeptor $R_1$ an eine Festphase gebunden vorliegt, während der andere Rezeptor $R_2$, der eine Markierung trägt, löslich ist. Es gibt noch weitere Varianten mit zwei löslichen Rezeptoren $R_1$, $R_2$ oder mit einem gebundenen Rezeptor $R_1$ und zwei löslichen Rezeptoren, von denen einer, $R_2$, eine Markierung trägt. Für alle diese Varianten ist das erfindungsgemäße Verfahren geeignet.

Als Rezeptoren $R_1$ und $R_2$ können z.B. Substanzen verwendet werden, die ein für den zu bestimmenden Antikörper spezifisches Epitop tragen. Dies können Antigene, Antigenfragmente, Antiidiotypantikörper oder Haptene sein. Die in den Rezeptoren $R_1$ und $R_2$ enthaltenen antikörperspezifischen Substanzen können identisch oder verschieden sein.

Die Markierung eines Rezeptors ist an sich bekannt. Üblicherweise werden Enzyme, radioaktive Substanzen, fluoreszierende oder chemolumineszierende Substanzen verwendet. Der Nachweis erfolgt dann in an sich bekannter Weise durch Messung der Radioaktivität, Chemilumineszenz oder Fluoreszenz oder durch die Enzymreaktion mit einem entsprechenden Farbstoffsubstrat.

Unter dem Rezeptor $R_S$ sind Substanzen zu verstehen, die mindestens ein für den zu bestimmenden Antikörper spezifisches Epitop tragen, wie z. B. Antigene, Antigenfragmente, Anti-idiotyp-Antikörper oder Haptene. Die erfindungsgemäß verwendeten, mit diesem Rezeptor $R_S$ bindefähigen Antikörper oder Antikörperfragmente umfassen auch Konjugate von Antikörpern oder Antikörperfragmenten mit Haptenen.

Bei der Durchführung eines Immunoassays zur Bestimmung eines Antikörpers wird nun erfindungsgemäß eine Kontrolle mitgeführt als interner Standard. Außerdem dient diese Kontrolle als Funktionskontrolle für alle im Test eingesetzten Reagenzien. Hierzu wird statt der Probe eine Standardlösung eingesetzt, die ein durch kovalente Verknüpfung hergestelltes Konjugat aus einem mit der für den zu bestimmenden Antikörper spezifischen antigenen Determinante bindefähigen, nicht-humanen Antikörper und einem Human-Immunglobulin enthält. Außer dem vollständigen Antikörper kann auch dessen Fab- oder F(ab')$_2$-Fragment eingesetzt werden. Statt des Human-Immunglobulins kann ebenso dessen Fc-Fragment verwen-

4

det werden. Alle Kombinationen zwischen den beiden Komponenten und deren Fragmenten sind dabei möglich.

Das Konjugat wird durch kovalente Bindung der beiden Komponenten - Antikörper und Human-Immunglobulin, bzw. deren Fragmente - hergestellt. Verfahren zur kovalenten Bindung von Proteinen sind wohlbekannt. Eine Vielzahl von Verfahren ist z. B. in P. Tijssen, Practice and Theory of Enzyme Immunoassays, Elsevier, Amsterdam, 1985, Chapter 11, beschrieben. Diese Verfahren sind gut geeignet zur Herstellung der erfindungsgemäß verwendeten Konjugate unter Verwendung homobifunktioneller oder heterobifunktioneller Reagenzien als Vernetzungsmittel.

Bevorzugt wird zur Herstellung des Konjugats in eine der beiden Komponenten eine Thiolgruppe und in die andere der beiden Komponenten eine Maleimidgruppe eingeführt. Die Konjugation wird dann erreicht durch kovalente Bindung des Thiolanteils mit der Maleimidfunktion.

Als Antikörper sind sowohl polyklonale als auch monoklonale Antikörper, die mit der für den zu bestimmenden Antikörper spezifischen antigenen Determinante bindefähig sind, geeignet. Bevorzugt werden jedoch monoklonale Antikörper verwendet aus einem mit der antigenen Determinante Antikörper bildenden Tier. Als Tierspezies ist z. B. das Meerschweinchen zu nennen; bevorzugt werden Antikörper aus Mäusen oder Ratten verwendet.

Der zu bestimmende Antikörper kann verschiedenen Antikörperklassen entstammen. Als Beispiel ist zu nennen IgG, z. B. bei der Diagnose von Infektionen; IgM im Frühstadium einer Infektion; IgA beim Nachweis spezieller Infektionen wie z. B. Infektionen mit dem Epstein-Barr-Virus und IgE, das bei Allergien auftritt.

Gegenstand der Erfindung ist weiterhin ein Kontrollreagenz für die Bestimmung eines Antikörpers in menschlichen Körperflüssigkeiten nach dem Prinzip des Immunoassays, das ein durch kovalente Verknüpfung hergestelles Konjugat aus einem mit der für den zu bestimmenden Antikörper spezifischen antigenen Determinante bindefähigen, nicht-humanen Antikörper oder dessen Fab- oder F(ab′)$_2$-Fragment und einem Human-Immunglobulin oder dessen Fc-Teil enthält.

Das erfindungsgemäße Kontrollreagenz wird für den jeweiligen speziellen Immunoassay hergestellt. Dabei wird jeweils spezifisch für jeden Test ein Antikörper oder dessen Fab- oder F(ab′)$_2$-Fragment mit einem Human-Immunglobulin oder dessen Fc-Teil konjugiert. Um größtmögliche Vergleichbarkeit mit den unbekannten Serumproben zu erreichen, wird das Kontrollreagenz bevorzugt in Normalserum, das gegebenenfalls noch Detergenzien enthält, gelöst. Aber auch die Lösung in serumfreiem Puffer ist möglich.

Erfindungsgemäß wird ein Verfahren und ein Kontrollreagenz für die Bestimmung eines Antikörpers in Körperflüssigkeiten zur Verfügung gestellt, das es ermöglicht, Testergebnisse miteinander zu vergleichen und interne Standards zur Erhöhung der Genauigkeit der Messung mitzuführen. Das erfindungsgemäß verwendete Konjugat ist in unbeschränkter Menge reproduzierbar herzustellen.

Die Erfindung wird durch die folgenden Beispiele erläutert:

**Beispiel 1**

Herstellung eines Konjugats aus Mäuse-monoklonalem Antikörper gegen Insulin und Human-Immun-γ-Globulin.

Um die beiden Komponenten - Insulinantikörper und Immun-γ-Globulin - zu vernetzen, wurde in den Antikörper eine Thiolgruppe und in das Immun-γ-Gloublin eine Maleimidgruppe eingeführt. Die Einführung der Thiolgruppe in den Mäuseantikörper wurde im wesentlichen durchgeführt, wie es bei Klotz und Heiney (Arch. Biochem. Biophys., 96 (1962) 605-612) beschrieben ist. Dazu wurden 10 mg Mäuse-Antiinsulin-Antikörper, ATCC HB123, in 1 ml einer 0,1 M Kaliumphosphatlösung mit einem pH von 8,2, die 150 mM NaCl enthielt, gelöst. 20 μl S-Acetylmercaptosuccinanhydrid (43,6 mg/ml Dimethylsulfoxid) wurden zu der obigen Lösung zugegeben und eine Stunde bei 25°C reagieren gelassen. Anschließend wurde die Reaktionsmischung bei 4°C gegen 0,1 M Kaliumphosphat, pH 6,2, das 1 mM EDTA enthielt, dialysiert.

30 mg Human-Immun-γ-Globulin wurden in 1 ml einer 30 mM Natriumphosphatlösung mit einem pH von 7 aufgelöst. 20 μl Maleimidohexanoyl-N-hydroxysuccinimidester (18,4 mg/ml Dimethylsulfoxid) wurden zugegeben und eine Stunde bei 25°C reagieren gelassen. Die Lösung wurde dann auf eine Ultrogel® AcA 202 Säule gegeben und die Fraktionen, die Protein enthielten, wurden gesammelt.

6 mg des Acetylmercaptosuccinilierten Mäuse-Antikörpers und 18 mg des Maleimidhuman-Immun-γ-Globulins wurden in einem Volumen von 5 ml 0,1 M Kaliumphosphatlösung mit einem pH von 7,0, die 1 mM EDTA und 20 mM Hydroxylamin enthielt, inkubiert. Das Hydroxylamin dient dabei dazu, die Thiolgruppen durch Spaltung der Thiolesterbindung zu aktivieren. Die Reaktionsmischung wurde 2 Stunden bei 25°C inkubiert. Die Inkubationsmischung wurde dann auf 1 ml durch Ultrafiltration konzentriert und auf eine Sephacryl®-S-300 (1,5x50cm)-Säule gegeben und mit 50 mM Natriumphosphat, enthaltend 150 mM NaCl und 0,1 % Natriumazid equilibriert. Die Fraktionen, die vor den monomeren Immun-γ-Globulinen eluierten, wurden gesammelt und enthielten das Konjugat aus Mäuse-Antiinsulin-Antikörper und Human-Immun-$\gamma$-Globulin.

**Beispiel 2**

Die Durchführung der Antikörperbestimmung erfolgte im wesentlichen, wie es bei Dean et al, (Diabetologia 29 (1986), 339-342) beschrieben ist.

Zur Herstellung von insulinbeschichteten Platten wurde Human-Insulin (1 $\mu$g/ml) in 50 mM Natriumcarbonatpuffer mit einem pH von 9,6 gelöst. Die Beschichtung (125 $\mu$l pro Napf) erfolgte während 5 Stunden bei 20°C in Flachbodenmikrotiterplatten (Nunc Immunoplate I). Danach wurden die Platten ausgekippt, auf Zellstoff ausgeklopft und mit 20 % (v/v) Ziegen-Normalserum in PBS (phosphate buffered saline) (150 $\mu$l pro Napf) über Nacht bei 20°C nachbeladen. Die Nachbeladelösung wurde ausgekippt, die Platten auf Zellstoff gründlich ausgeklopft, über Nacht bei Raumtemperatur getrocknet, anschließend mit Klebefolie versiegelt und bis zum Gebrauch bei -20°C aufbewahrt.

Mit diesen Platten wurde dann ein Enzymimmunoassay durchgeführt. Die Probeninkubation erfolgt dabei in 10% (v/v) Ziegen-Normalserum/5% (v/v) Tween® 20 in PBS während 1,5 Stunden bei Raumtemperatur unter ständigem Schütteln (300 rpm). Die entsprechende Probenverdünnung wurde als Doppelwert (100 $\mu$l pro Napf) inkubiert. Die Platte wurde nach 1,5 Stunden abgesaugt und dreimal mit je 300 $\mu$l Waschmedium pro Napf (0,1 % (v/v) Tween® 20 in PBS) bei Standzeiten von 1/3/3 Minuten gewaschen.

Die gebundenen Insulin-Antikörper-Moleküle wurden mit einem Zweitantikörper aus Schaf, der gegen den Fc$\gamma$-Teil des humanen Immunglobulin-Moleküls gerichtet und seinerseits mit Peroxidase markiert war, nachgewiesen. Die zweite Immunreaktion dauerte eine Stunde (ebenfalls mit Schütteln bei 300 rpm) und wurde mit einem, wie oben beschriebenen, Waschschritt abgeschlossen.

Die Indikatorreaktion der Peroxidase erfolgte in Citratpuffer bei pH 4,5, wobei die Peroxidase mit Natriumperborat und ABTS ® reagierte und die Farbentwicklung zu den angegebenen Zeiten am ELISA-Photometer bei 405 nm (abzüglich des Signals bei der Referenzwellenlänge 490 nm) mit ABTS ® -Lösung als Leerwert gemessen wurde.

Tabelle I zeigt die Probenauswertung zu verschiedenen Zeiten nach dem Start der Substratreaktion. Es ergibt sich, daß das Verhältnis der Meßwerte von Probe und Funktionskontrolle während der gesamten Substratreaktionszeit konstant bleibt. Damit kann zu beliebigen Zeiten nach dem Start der Substratreaktion gemessen werden.

EP 0 291 086 B1

## T a b e l l e   I

| Probe | Substratreaktion (min) | | | | | | | |
|---|---|---|---|---|---|---|---|---|
| | 15 | | 30 | | 45 | | 60 | |
| | $\Delta$E | % | $\Delta$E | % | $\Delta$E | % | $\Delta$E | % |
| MAK ⟨Insulin⟩-H-IgG (1:30) | 0,576 | 100 | 0,962 | 100 | 1,276 | 100 | 1,534 | 100 |
| 1 | 0,057 | 9,9 | 0,097 | 10,1 | 0,122 | 9,6 | 0,151 | 9,8 |
| 2 | 0,155 | 26,9 | 0,261 | 27,1 | 0,349 | 27,4 | 0,427 | 27,8 |
| 3 | 0,330 | 57,3 | 0,560 | 58,2 | 0,756 | 59,2 | 0,902 | 58,8 |
| 4 | 0,338 | 58,7 | 0,570 | 59,3 | 0,776 | 60,8 | 0,946 | 61,7 |
| 5 | 0,506 | 87,8 | 0,849 | 88,3 | 1,127 | 88,3 | 1,369 | 89,2 |

Probenauswertung nach verschiedenen Subtrat-Reaktionszeiten (Meßwellenlänge 405 nm).

Tabelle II zeigt die Probenauswertung an verschiedenen Tagen. Auch hierbei zeigt sich, daß - trotz unterschiedlichem $\Delta$E-Wert für die Funktionskontrolle - das Verhältnis der Meßwerte von Probe und Funktionskontrolle konstant bleibt.

7

Tabelle    II

| Tag | MAK ⟨Ins⟩ - H-IgG | | Probe | | |
|---|---|---|---|---|---|
| | $\Delta$ E | % | 1 % | 2 % | 3 % |
| 1 | 1,235 | 100 | 33,7 | 32,5 | 64,7 |
| 2 | 1,237 | 100 | 39,0 | 33,6 | 67,7 |
| 3 | 1,268 | 100 | 39,4 | 30,5 | 67,5 |
| 4 | 1,280 | 100 | 33,4 | 30,9 | 63,8 |
| 5 | 1,327 | 100 | 40,2 | 29,9 | 65,2 |
| 6 | 1,350 | 100 | 36,1 | 34,7 | 68,6 |
| 7 | 1,419 | 100 | 36,4 | 35,7 | 63,9 |

**Beispiel 3**

Herstellung eines Konjugats aus einem Fab-Fragment eines polyklonalen Anti-Insulin-Antikörpers mit einem humanen Fc $\gamma$-Fragment.

Aus dem polyklonalen Antikörper, Herstellung wie in DE 27 44 835 beschrieben, wird ein Fab-Fragment abgespalten, eine Thiolgruppe eingeführt, und wie ebenfalls in Beispiel 1 beschrieben, mit einem Fc $\gamma$-Fragment von Human-IgG gekoppelt. Die Methoden zur Antikörperspaltung, Aktivierung und Kopplung sind in P. Tijssen, Practice and theory of enzyme immunoassays (1985) Elsevier, Amsterdam, beschrieben.

**Beispiel 4**

Kontrolle des Insulin-Antikörpertests mit Hilfe eines Konjugats aus dem Fab-Fragment eines polyklonalen Insulinantikörpers aus Meerschweinchen und einem human Fc $\gamma$-Fragment.

Die Antikörperbestimmung wird, wie in Beispiel 2 beschrieben, durchgeführt.

Die Ergebnisse sind in Tabelle III enthalten.


## T a b e l l e   III

| Probe | Substratreaktionszeit (min) | | | | | |
|---|---|---|---|---|---|---|
| | 20 | | 40 | | 60 | |
| | $\Delta E$ | % | $\Delta E$ | % | $\Delta E$ | % |
| PAK⟨Insulin⟩- MS-Fab-H-Fcγ | 0,388 | 100 | 0,672 | 100 | 0,918 | 100 |
| Serum 1 | 0,052 | 13,4 | 0,092 | 13,7 | 0,126 | 13,7 |
| Serum 2 | 0,097 | 25,0 | 0,168 | 25,0 | 0,231 | 25,2 |
| Serum 3 | 0,254 | 65,5 | 0,437 | 65,0 | 0,595 | 64,8 |
| Serum 4 | 0,378 | 97,4 | 0,657 | 97,8 | 0,894 | 97,4 |

Es zeigt sich, daß die Proben zu allen drei gemessenen Zeiten der Substratreaktion in konstantem Verhältnis zur Funktionskontrolle stehen. Die Meßzeit kann also frei gewählt werden.

**Beispiel 5**

Herstellung eines Konjugates aus polyklonalem Antikörper gegen menschliches Erythropoietin (EPO) und menschlichem Immunglobulin, sowie die Bestimmung von EPO-Antikörpern
Weiße Neuseeland-Kaninchen wurden mit 50 μg Human-EPO immunisiert und am Tag 7, 14, 30, dann alle 30 Tage nach Erstimmunisierung mit jeweils 25 μg Antigen/Tier nachgeimpft. Zusätzlich wurde immer komplettes Freundsches Adjuvans gegeben. Aus dem Serum der Tiere wurde mittels Ammoniumsulfatfällung und Austauschchromatographie IgG gereinigt. Dieses wurde anschließend wie in Beispiel 1 beschrieben an menschliches IgG gekoppelt. Dieses Konjugat dient als Kontrollreagenz für die Bestimmung von humanen EPO-Antikörpern.
Die Durchführung der Antikörperbestimmung erfolgte wie in Beispiel 2 beschrieben, außer daß die Mikrotiterplatte mit 1,25 μg EPO/ml beschichtet wurde.
Auch hier ergibt sich (siehe Tabelle IV) ein konstanter Quotient der Meßsignale von Probe und Kontrollreagenz, so daß die Farbentwicklung zu einem beliebigen Zeitpunkt zwischen 15 und 60 min nach Beginn der Substratreaktion gemessen und ausgewertet werden kann.

EP 0 291 086 B1

T a b e l l e   IV

| Probe | Substratreaktionszeit (min) | | | | | | | |
|---|---|---|---|---|---|---|---|---|
| | 15 | | 30 | | 45 | | 60 | |
| | $\Delta E$ | % | $\Delta E$ | % | $\Delta E$ | % | $\Delta E$ | % |
| PAK⟨EPO⟩ KanIgG-HIgG | 0,354 | 100 | 0,632 | 100 | 0,848 | 100 | 0,975 | 100 |
| Probe 1 | 0,049 | 13,8 | 0,085 | 13,4 | 0,111 | 13,1 | 0,128 | 13,1 |
| Probe 2 | 0,085 | 24,0 | 0,151 | 23,9 | 0,201 | 23,7 | 0,230 | 23,6 |
| Probe 3 | 0,120 | 33,9 | 0,214 | 33,9 | 0,284 | 33,5 | 0,328 | 33,6 |
| Probe 4 | 0,166 | 46,9 | 0,298 | 47,2 | 0,392 | 46,2 | 0,450 | 46,2 |

**Patentansprüche**

1. Verfahren zur Bestimmung eines Antikörpers in menschlichen Körperflüssigkeiten nach dem Prinzip des Immunoassays, wobei man eine den zu bestimmenden Antikörper enthaltende Probe mit mindestens zwei verschiedenen Rezeptoren $R_1$, $R_2$, von denen ein Rezeptor $R_1$ eine für den zu bestimmenden Antikörper spezifische antigene Determinante trägt und ein Rezeptor $R_2$ eine Markierung trägt, inkubiert, den Teil, der die gebundene Markierung enthält von dem Teil, der die ungebundene Markierung enthält, trennt und die Markierung in einem der beiden Teile mißt,
**dadurch gekennzeichnet,** daß man zur Kontrolle statt der Probe eine Standardlösung einsetzt, die ein durch kovalente Verknüpfung hergestelltes Konjugat aus einem mit dem für den zu bestimmenden Antikörper spezifischen Rezeptor $R_S$ bindefähigen nicht-humanen Antikörper oder dessen Fab- oder F-(ab')$_2$-Fragment und einem Humanimmunglobulin oder dessen Fc-Teil enthält.

2. Verfahren nach Anspruch 1, **dadurch gekennzeichnet,** daß als Antikörper eine monoklonaler Antikörper eingesetzt wird.

3. Verfahren nach Anspruch 2, **dadurch gekennzeichnet,** daß ein monoklonaler Antikörper aus einem mit dem Rezeptor $R_S$ Antikörper bildenden Tier, insbesondere aus Mäusen oder Ratten verwendet wird.

4. Verfahren nach Anspruch 1, **dadurch gekennzeichnet,** daß Auto-Antikörper oder nach einer Infektion, bei einer Therapie oder infolge einer Allergie gebildete Antikörper bestimmt werden.

5. Verfahren nach Anspruch 1, **dadurch gekennzeichnet,** daß Auto-Insulin-Antikörper, Inselzellen-Antikörper, Hepatitis-Antikörper, Erythropoietin-Antikörper, Plasminogenaktivator-Antikörper und HIV-Antikörper in menschlichen Körperflüssigkeiten bestimmt werden.

6. Kontrollreagenz für die Bestimmung eines Antikörpers in menschlichen Körperflüssigkeiten nach dem Prinzip des Immunoassays, enthaltend ein Konjugat aus einem mit dem für den zu bestimmenden

10

Antikörper spezifischen Rezeptor $R_S$ bindefähigen Antikörper oder dessen Fab- oder $F(ab')_2$-Fragment und einem Human-Immunglobulin oder dessen Fc-Teil.

7. Kontrollreagenz nach Anspruch 6, **dadurch gekennzeichnet,** daß er ein Konjugat aus einem Antikörper gegen Insulin oder dessen Fab- oder $F(ab')_2$-Fragment und einem Human-Immunglobulin oder dessen Fc-Teil enthält.

**Claims**

1. Process for the determination of an antibody in human body fluids according to the immunoassay principle whereby one incubates a sample containing the antibody to be determined with at least two different receptors $R_1$, $R_2$, of which one receptor $R_1$ carries an antigenic determinant specific for the antibody to be determined and one receptor $R_2$ carries a labelling, separates the part which contains the bound labelling from the part which contains the unbound labelling and measures the labelling in one of the two parts, characterised in that, for the control, instead of the sample, one uses a standard solution which contains a conjugate, prepared by covalent linkage, of a non-human antibody bindable with the receptor $R_s$ specific for the antibody to be determined or its Fab or $F(ab')_2$ fragment and a human immunoglobulin or its Fc part.

2. Process according to claim 1, characterised in that a monoclonal antibody is used as antibody.

3. Process according to claim 2, characterised in that a monoclonal antibody is used from an animal forming antibodies with the receptor $R_3$, especially from mice or rats.

4. Process according to claim 1, characterised in that auto-antibodies or antibodies formed after an infection, in the case of a therapy or as a result of an allergy are determined.

5. Process according to claim 1, characterised in that auto-insulin antibodies, islet cell antibodies, hepatatitis antibodies, erythropoietin antibodies, plasminogen activator antibodies and HIV antibodies are determined in human body fluids.

6. Control reagent for the determination of an antibody in human body fluids according to the immunoassay principle, containing a conjugate , produced by covalent linking, of a non-human antibody bindable with the receptor $R_3$ specific for the antibody to be determined or its Fab or $F(ab')_2$ fragment with a human immunoglobulin or its Fc part.

7. Control reagent according to claim 6, characterised in that it contains a conjugate of an antibody against insulin or its Fab or $F(ab')_2$ fragment and a human immunoglobulin or its Fc part.

**Revendications**

1. Procédé pour déterminer un anticorps dans les fluides corporels humains selon le principe de l'immunoanalyse, dans lequel on incube un échantillon contenant l'anticorps à déterminer avec au moins deux récepteurs différents $R_1$, $R_2$ parmi lesquels un récepteur $R_1$ porte un déterminant antigénique spécifique de l'anticorps à déterminer et un récepteur $R_2$ porte un marquage, on sépare la partie qui contient le marquage lié de la partie qui contient le marquage non lié et on mesure le marquage dans l'une des deux parties, caractérisé en ce que l'on utilise pour le contrôle à la place de l'échantillon une solution standard qui contient un conjugué, préparé par liaison covalente, constitué par un anticorps non humain, ou son fragment Fab ou $F(ab')_2$, capable de se lier au récepteur $R_S$ spécifique de l'anticorps à déterminer et par une immunoglobuline humaine ou sa partie Fc.

2. Procédé selon la revendication 1, caractérisé en ce que l'on utilise comme anticorps un anticorps monoclonal.

3. Procédé selon la revendication 2, caractérisé en ce que l'on utilise un anticorps monoclonal provenant d'un animal formant des anticorps avec le récepteur $R_S$, en particulier de souris ou de rats.

**4.** Procédé selon la revendication 1, caractérisé en ce que l'on détermine des auto-anticorps ou des anticorps formés à la suite d'une infection, lors d'une thérapie ou par suite d'une allergie.

**5.** Procédé selon la revendication 1, caractérisé en ce que l'on détermine des auto-anticorps anti-insuline, des anticorps anti-cellules insulaires, des anticorps anti-hépatite, des anticorps anti-érythropoïétine, des anticorps anti-activateur du plasminogène et des anticorps anti-HIV dans les fluides corporels humains.

**6.** Réactif de contrôle pour la détermination d'un anticorps dans les fluides corporels humains selon le principe de l'immuno-analyse contenant un conjugué, préparé par liaison covalente, constitué par un anticorps non humain, ou son fragment Fab ou $F(ab')_2$, capable de se lier spécifiquement au récepteur $R_S$ spécifique de l'anticorps à déterminer et par une immunoglobuline humaine ou sa partie Fc.

**7.** Réactif de contrôle selon la revendication 6, caractérisé en ce qu'il contient un conjugué constitué par un anticorps contre l'insuline, ou son fragment Fab ou $F(ab')_2$, et par une immunoglobuline humaine ou sa partie Fc.